# FASCICULE DE BREVET EUROPEEN

(11) **EP 1 546 592 B1**
(45) Date de publication et mention de la délivrance du brevet: **03.03.2010**
(21) Numéro de dépôt: 03775467.8
(22) Date de dépôt: 03.10.2003
(51) Int. Cl.: F16K 35/04

(54) **ROBINET A CLE TOURNANTE INDEXABLE**
VENTIL MIT INDEXIERBARES SCHLIESSELEMENT
COCK COMPRISING INDEXABLE ROTATING KEY

(30) Priorité: 04.10.2002 FR 0212335
(43) Date de publication de la demande: 29.06.2005
(73) Titulaire: VYGON, F-95440 Ecouen (FR)
(72) Inventeur: CARREZ, Jean Luc, F-95440 Ecouen (FR); DALLE, Valéry, F-60270 Gouvieux (FR); LESIMPLE, Laurent, F-93290 Tremblay-en-France (FR); HUET, Jean-Max, F-92110 Clichy (FR)
(74) Mandataire: Texier, Christian
(86) Numéro de dépôt international: PCT/FR2003/002910
(87) Numéro de publication internationale: WO 2004/031635

(56) Documents cités:
- EP-A- 0 733 836
- DE-C- 492 954
- GB-A- 1 357 424
- US-A- 3 783 900
- US-A1- 2001 025 942
- PATENT ABSTRACTS OF JAPAN vol. 2002, no. 09, 4 septembre 2002 (2002-09-04) & JP 2002 153562 A (JMS CO LTD), 28 mai 2002 (2002-05-28)

## Description

L'invention concerne un robinet à clé tournante indexable.

Elle concerne plus précisément un robinet à clé tournante dont la clé tourne dans un logement tubulaire d'un boisseau où la clé a été introduite axialement par une extrémité du logement jusque dans une position axiale déterminée par des moyens d'arrêt, la paroi latérale du logement comportant des passages qui débouchent dans une zone de logement par des ouvertures tandis que la clé est conçue avec un ou des passages pour obturer ces ouvertures ou établir une communication entre certaines ouvertures en fonction des positions de la clé au cours d'une rotation de la clé, le robinet comportant dans une autre zone du logement des moyens (dits moyens d'indexage) pour indiquer tactilement à l'opérateur que la clé est arrivée dans une position de service où elle établit une communication, ces moyens d'indexage comprenant des ergots et des encoches répartis sur des faces en regard de la clé et du logement en sorte qu'au cours d'une rotation de la clé les ergots pénètrent dans les encoches lors que la clé arrive dans une position de service et ne peuvent en ressortir que par un effort sensible exercé sur la clé pour la faire tourner, la clé ou le boisseau étant conçus de façon à permettre aux ergots de ressortir des encoches par effet élastique en conséquence de cet effort.

Un exemple d'un tel robinet est décrit dans le brevet US 5 832 959.

Dans cet exemple, le boisseau et la clé sont tous deux en matériau élastique pour réaliser l'effet élastique requis pour que les ergots puissent sortir des encoches.

Une telle élasticité est préjudiciable à l'étanchéité de la zone où débouchent les passages du boisseau, d'autant que les moyens d'indexage sont situés dans une zone du logement dont le diamètre interne est supérieur à celui de la zone où débouchent des passages du boisseau.

La publication US 2001/0025942 décrit un robinet dont le logement du boisseau comporte un fond fermé qui présente un relief déterminant entre lui et la paroi latérale élastique du logement une gorge annulaire dans laquelle peut tourner une couronne formée à l'extrémité de la clé, les ergots et les encoches étant formés sur le dit relief et sur ladite couronne.

Un but de l'invention est de fournir un robinet à étanchéité améliorée et dont l'élasticité ne perturbe pas l'étanchéité requise dans la zone des ouvertures.

On y parvient selon l'invention avec un robinet dans lequel le logement comporte un fond fermé qui présente un relief lequel détermine entre lui et la paroi latérale du logement une gorge annulaire et la clé présente à son extrémité une couronne élastiquement déformable dans un plan transversal à l'axe de rotation de la clé qui tourne dans cette gorge, les ergots et les encoches étant formés sur ledit relief et sur ladite couronne, et la zone où se trouvent les moyens d'indexage ayant un diamètre réduit par rapport à la zone où débouchent les ouvertures des passages de la paroi latérale du logement du boisseau.

Dans une réalisation préférée, ladite couronne est divisée en secteurs en arc qui sont séparés par des coupures et dans lesquels sont formés les ergots ou les encoches.

Le robinet de l'invention peut encore présenter une ou plusieurs des autres caractéristiques suivantes :
- Ladite couronne est divisée en deux secteurs identiques qui comportent chacun un ergot ou une encoche.
- Ladite couronne est divisée en quatre secteurs identiques qui comportent chacun un ergot ou une encoche.
- Ledit relief est conformé latéralement pour former huit encoches ou huit ergots.
- La zone où se trouvent les moyens d'indexage a un diamètre réduit par rapport à la zone où débouchent lesdites ouvertures.
- Le logement du boisseau a une section droite qui diminue depuis l'extrémité d'introduction de la clé et la clé a une forme générale tronconique.
- Ledit relief a un profil régulier constitué d'une succession de secteurs convexes alternant avec des secteurs concaves constituant lesdites encoches.
- La clé est arrêtée axialement dans le logement par pénétration d'une nervure circulaire formée sur la clé dans une gorge formée dans la paroi latérale du logement.

On décrira ci-après des exemples de réalisation d'un robinet selon l'invention en référence aux figures du dessin joint sur lequel :
- la figure 1 est une vue en perspective de la clé et du boisseau d'un robinet avant introduction de la clé dans le boisseau, une moitié seulement de la clé et du boisseau étant représentés sur la figure ;
- la figure 2 est une vue en perspective du boisseau seul avec arrachement pour faire apparaître l'intégralité du relief formé sur le fond du logement;
- la figure 3 est une vue en perspective de la clé seule représentée sens dessus dessous pour faire apparaître l'intégralité de sa couronne ;
- la figure 4 est une perspective en vue de dessous d'une coupe transversale du robinet dans la région de la couronne et du relief ;
- les figures 5 à 7 sont des vues relatives à une variante de réalisation du robinet qui correspondent respectivement aux figures 1, 3 et 4 ;
- la figure 8 est un schéma illustrant les possibilités de communication en soi connues d'un robinet à trois voies,et
- les figures 9 et 10 sont des sections droites d'un robinet dont la clé a huit positions d'arrêt, respectivement dans un état où la clé est dans une position d'arrêt et dans un état intermédiaire entre deux positions d'arrêt.

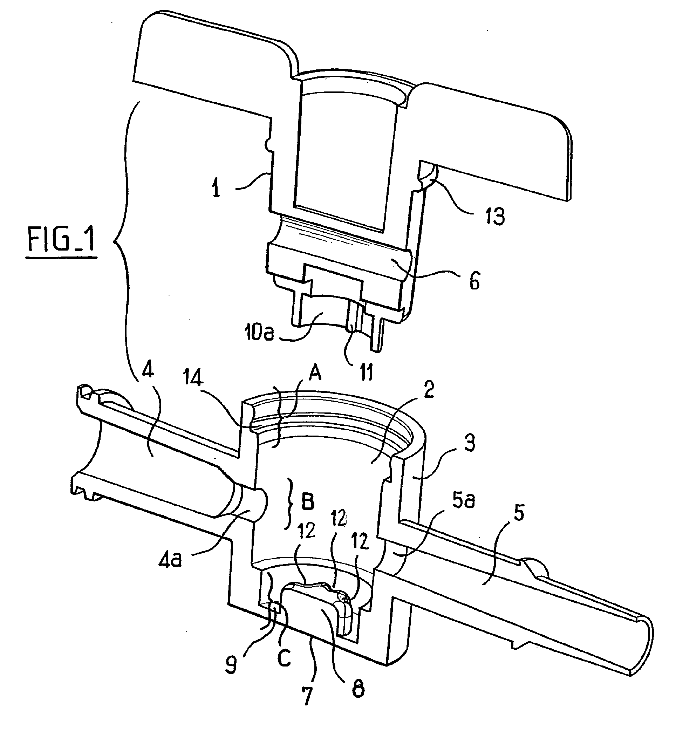

La figure 1 représente la clé (1) et le boisseau (2) d'un robinet, avant introduction de la clé dans le boisseau par une extrémité du logement (3). Pour simplifier la figure, on a supposé que le boisseau ne comporte que deux passages (4, 5) qui débouchent dans le logement par des ouvertures (4a, 5a) et que la clé ne comporte qu'un passage (6) pour mettre en communication les dites ouvertures.

Conformément à l'invention, le logement (3) du boisseau (2) présente un fond fermé (7) muni d'un relief (8) qui détermine entre lui et la paroi latérale du logement une gorge annulaire (9), et la clé (1) présente à son extrémité une couronne (10) apte à tourner dans ladite gorge lorsque la clé est dans sa position d'arrêt axial.

Le profil du relief (8) formé sur le fond du logement du boisseau détermine une alternance régulière de secteurs convexes (8a) et de secteurs concaves (8b). Les secteurs concaves (8b) constituent les encoches.

Dans l'exemple représenté, le relief (8) détermine ainsi huit encoches (12).

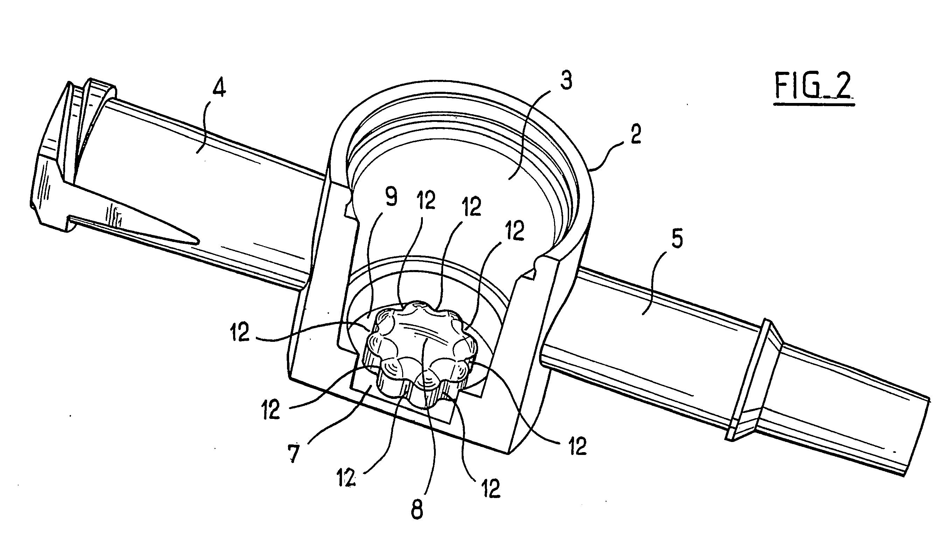

Le relief (8) dont une moitié seulement apparaît sur la figure 1 est visible dans son intégralité sur la figure 2.

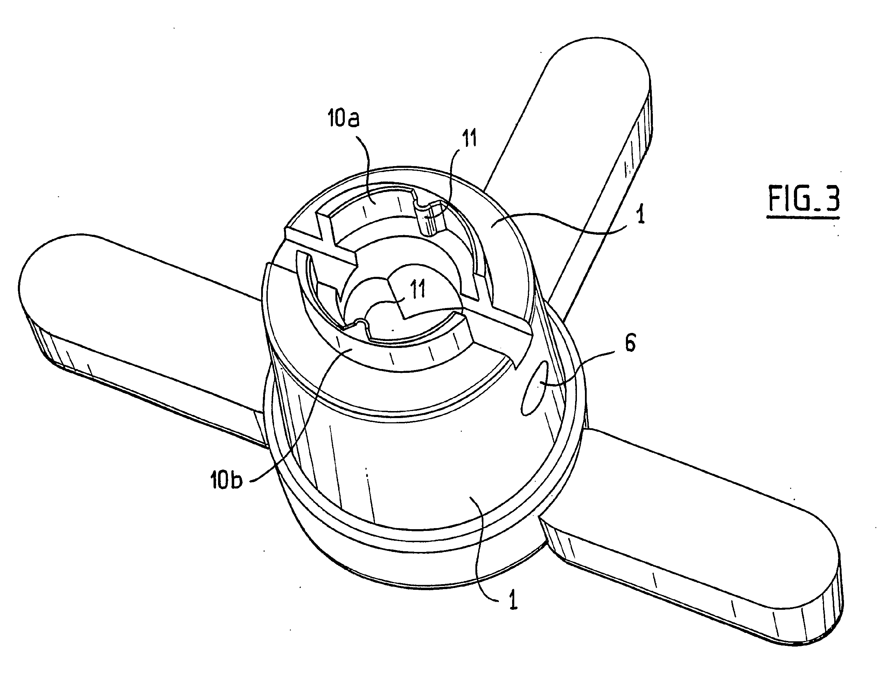

La couronne (10) de la clé est constituée dans cet exemple de deux secteurs identiques (10a, 10b) en forme d'arc séparés par des coupures et qui présentent chacun un ergot vertical (11) en relief. Un seul secteur apparaît sur la figure 1 mais les deux secteurs sont visibles dans son intégralité sur la figure 3 où la clé est représentée sens dessus dessous.

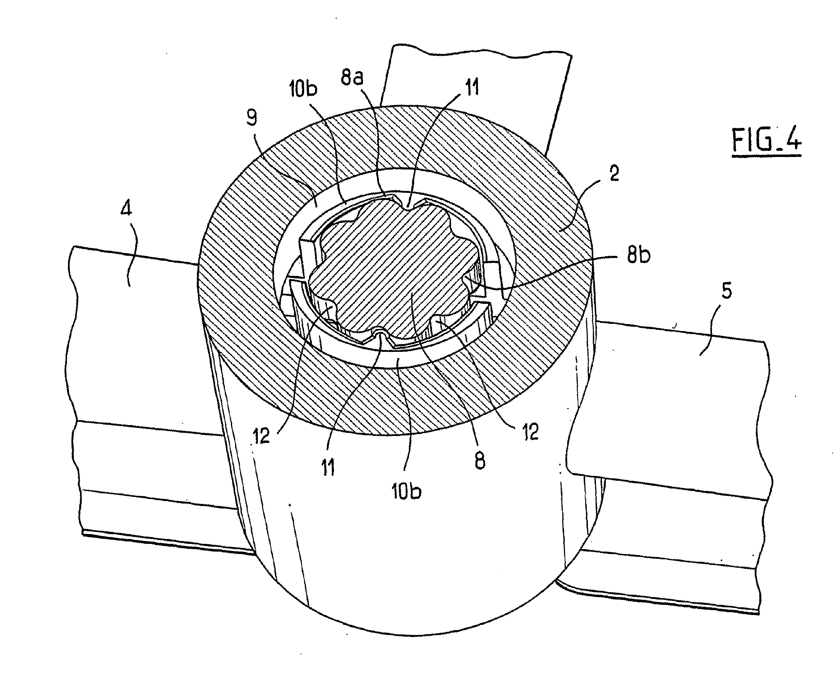

L'interaction de la couronne et du relief est clairement visible sur la figure 4. Sur cette figure, les parties hachurées sont des parties du boisseau.

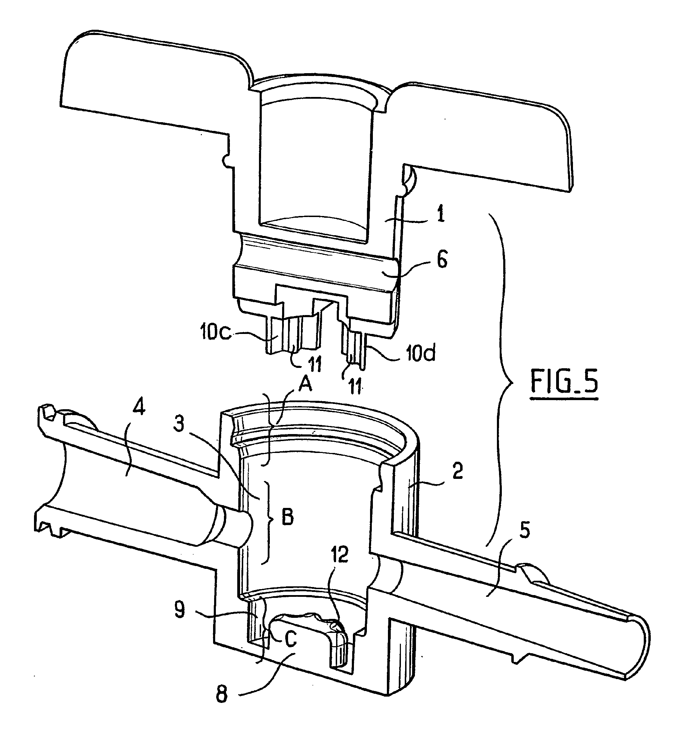

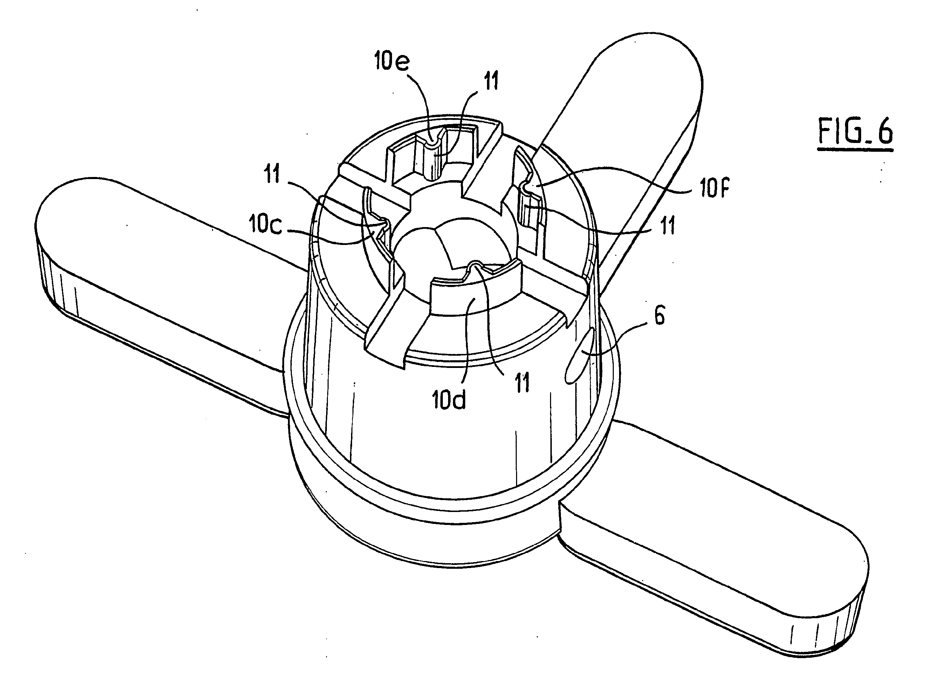

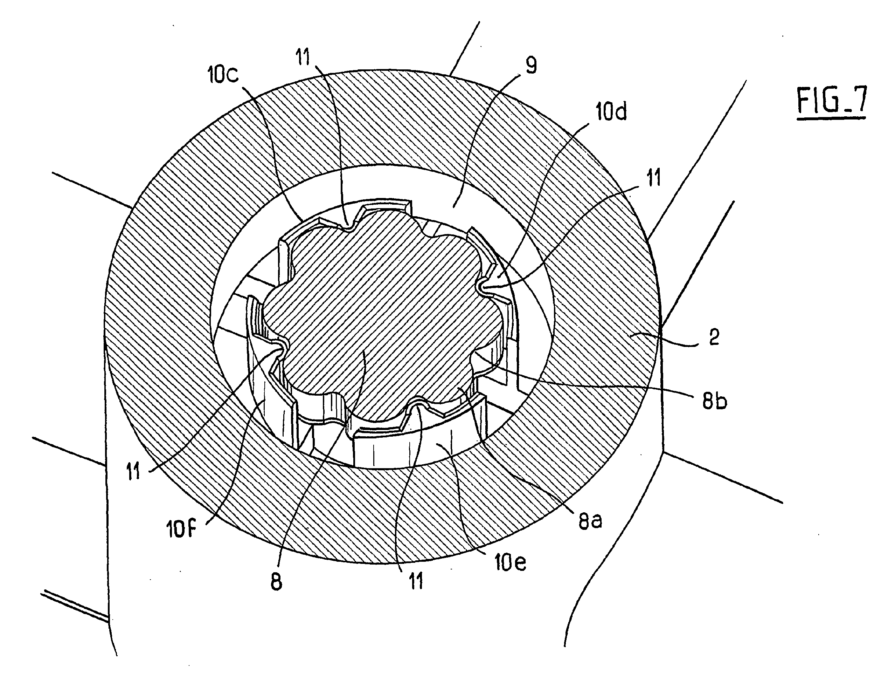

On a représenté sur les figures 5 à 7 qui correspondent respectivement aux figures 1, 3 et 4 une variante de réalisation où la couronne de la clé est coupée en quatre secteurs séparés en forme d'arc (10c, 10d, 10e, 10f) qui portent chacun un ergot. Le boisseau peut être identique à celui de la réalisation précédente.

L'invention n'est pas limitée à ces réalisations données à titre d'exemple. Il est évident que le nombre des secteurs de la couronne, le nombre d'ergots et le nombre d'encoches peuvent varier selon les réalisations.

La sensation effective de l'indexage peut être réglée par la hauteur des arcs de la couronne, la longueur des arcs, le nombre des arcs, la hauteur de la forme en relief au fond du logement.

La position axiale de la clé dans le boisseau est déterminée, par exemple comme dans le cas représenté, par clippage d'une nervure annulaire extérieur (13) de la clé dans une gorge (14) de la paroi annulaire du boisseau.

On voit également sur les figures que la zone (C) où se trouvent les moyens d'indexage a un diamètre réduit par rapport à la zone (B) où débouchent des perçages du boisseau.

Conformément à une caractéristique de l'invention, les zones (A) de clippage, (B) de communication et (C) d'indexage sont étagées et ont des sections droites qui diminuent d'une zone à la suivante.

Il va de soi que l'invention n'est pas limitée à une seule possibilité de communication et que l'on peut réaliser toutes les possibilités de communication habituelles avec un robinet conforme à l'invention, en faisant varier le nombre de passages, de façon en soi connue.

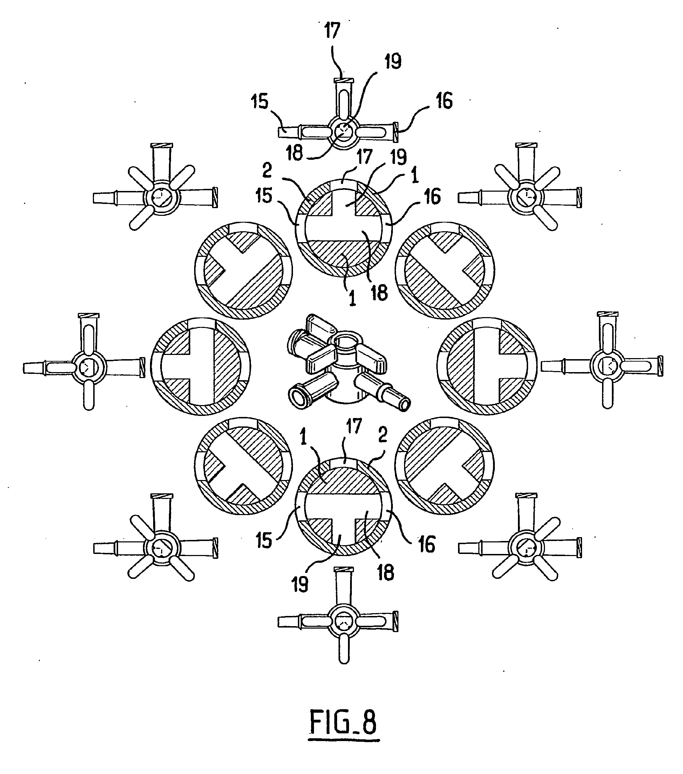

Pour mémoire, on a schématisé sur la figure 8 un robinet à trois voies avec ses différentes possibilités de mise en communication. Le boisseau présente trois passages 15, 16, 17 disposés en T et la clé présente deux passages 18, 19 perpendiculaires.

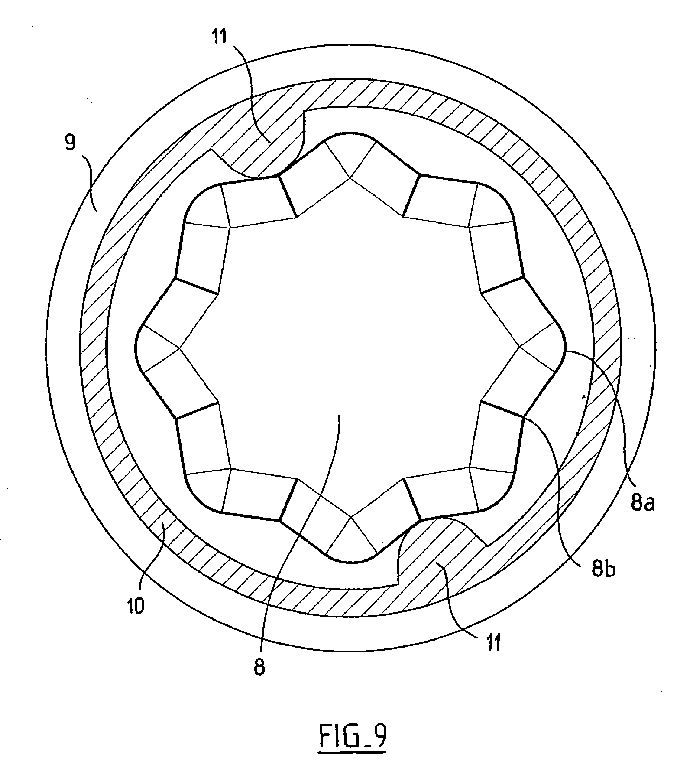

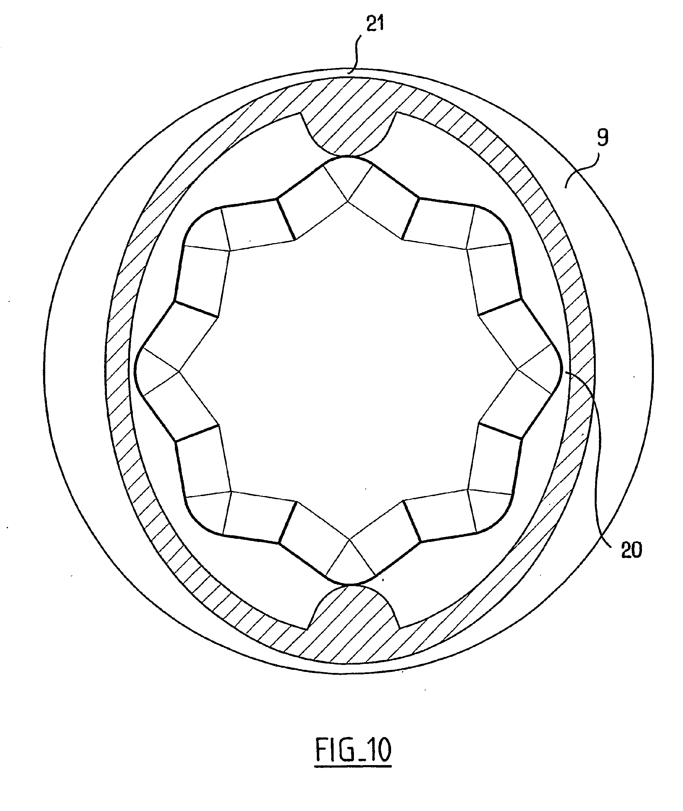

Sur les schémas des figures 9 et 10, on a représenté un robinet qui présente un relief octogonal (8) formé sur la paroi de fond du logement du boisseau (21), avec ses parties convexes (8a) et ses parties concaves (8b), et le bout de la clé constitué par une couronne cylindrique déformable (10)à paroi mince déformable transversalement qui forme deux godrons (11) diamétralement opposés.

Sur la figure 9, les deux godrons sont rapprochés diamétralement l'un de l'autre contre deux concavités opposées du relief.

Sur la figure 10 ou la couronne est déformée élastiquement du fait que les godrons sont montés sur les convexités du relief, ce qui déforme élastiquement la paroi de la couronne de la clé selon une ellipse, il reste un jeu (20) entre les convexités (8a) du relief et la couronne (10), et un jeu (21) entre la couronne (10) et la paroi (9) du logement du boisseau située en vis-à-vis.

La déformation élastique de cercle à ellipse de la clé assure un rôle de ressort pour ramener les godrons dans les zones (8b) en assurant un indexage précis, même après de nombreuses utilisations de la clé .

Le robinet de l'invention est destiné notamment à l'utilisation dans l'appareillage médical.

L'invention n'est pas limitée aux réalisations qui ont été décrites comme exemples préférés.

## Revendications

1. Robinet à clé tournante dont la clé (1) tourne dans un logement tubulaire (3) d'un boisseau (2) où la clé a été introduite axialement par une extrémité du logement jusque dans une position d'axiale déterminée par des moyens d'arrêt (13, 14), la paroi latérale du logement comportant des passages (4, 5) qui débouchent dans une zone (B) du logement par des ouvertures tandis que la clé est conçue avec un ou des passages (6) pour obturer ces ouvertures ou établir une communication entre certaines ouvertures en fonction de positions de services de la clé au cours d'une rotation de la clé, le robinet comportant dans une autre zone (C) du logement des moyens, dits moyens d'indexage, pour indiquer tactilement à l'opérateur que la clé est arrivée dans une position de service où elle établit une communication, ces moyens d'indexage comprenant des ergots (11) et des encoches (12) répartis sur des faces en regard de la clé et du logement en sorte qu'au cours d'une rotation de la clé les ergots pénètrent dans les encoches lorsque la clé arrive dans une position de service et ne peuvent en ressortir que par un effort sensible exercé sur la clé pour la faire tourner, la clé ou le boisseau étant conçus de façon à permettre aux ergots de ressortir par effet élastique des encoches en conséquence de cet effort, le logement (3) comportant un fond (7) fermé qui présente un relief (8) déterminant entre lui et la paroi latérale du logement une gorge annulaire (9), en la clé (3) présentant à son extrémité une couronne (10) qui tourne dans cette gorge, les ergots (11) et les encoches (12) étant formés sur ledit relief et sur ladite couronne (10),**caractérisé en ce que** ladite couronne (10) est élastiquement déformable dans un plan transversal à l'axe de rotation de la clé, et **en ce que** la zone(C) où se trouvent les moyens d'indexage a un diamètre réduit par rapport à la zone (B) où débouchent les dites ouvertures.

2. Robinet selon la revendication 1 dont ladite couronne (10) est divisée en secteurs en arc qui sont séparés par des coupures (13) et dans lesquels sont formés les ergots ou les encoches.

3. Robinet selon la revendication 2 dont ladite couronne (10) est divisée en deux secteurs (10a, 10b) identiques qui comportent chacun un ergot (11).

4. Robinet selon la revendication 2 dont ladite couronne est divisée en quatre secteurs identiques (10c, 10d, 10e, 10f) qui comportent chacun un ergot (11).

5. Robinet selon la revendication 3 ou 4 dont ledit relief (8) est conformé latéralement pour former huit encoches (12).

6. Robinet selon l'une des revendications 1 à 5 dont le logement (3) a une section droite qui diminue depuis ladite extrémité du logement et la clé (1) a une forme générale tronconique.

7. Robinet selon l'une des revendications 1 à 6 dans lequel ledit relief (8) a un profil régulier constitué d'une succession de secteurs convexes (8a) alternant avec des secteurs concaves (8b) constituant lesdites encoches.

8. Robinet selon l'une des revendications 1 à 7 dont la clé (1) est arrêtée axialement dans le logement (3) par pénétration d'une nervure annulaire (13) formée sur la clé dans une gorge (14) formée dans la paroi latérale du logement.

9. Robinet selon l'une des revendications précédentes dont la couronne a une section droite qui est circulaire à l'état non déformé et qui devient elliptique à l'état déformé élastiquement.

10. Robinet selon l'une des revendications précédentes dans lequel, lorsque la couronne (10) est déformée élastiquement, il existe un jeu (20) entre les convexités (8a) du relief (8) et la couronne et un jeu (21) entre la couronne et la paroi du logement du boisseau qui est en vis-à-vis de la couronne.

## Claims

1. A cock comprising a rotating key, the key (1) of which rotates in a tubular housing (3) of a barrel (2) where the key has been axially introduced through an end of the housing up into an axial position determined by blocking means (13, 14), the side wall of the housing including passages (4, 5) which open into an area (B) of the housing through apertures while the key is designed with passage (s) (6) for blocking these apertures or establishing a communication between certain apertures according to service positions of the key during a rotation of the key, the cock including in another area (C) of the housing, means, so-called indexing means, in order to tactilely tell the operator that the key has arrived in a service position where it establishes a communication, these indexing means comprising stubs (11) and notches (12) distributed over faces facing the key and the housing so that during a rotation of the key, the stubs penetrate the notches when the key arrives in a service position and may only emerge therefrom when a substantial force is exerted on the key to rotate it, the key or the barrel being designed in order to allow the stubs to emerge from the notches by an elastic effect resulting from this force, the housing (3) including a closed base (7) which has a raised element (8) determining between it and the side wall of the housing, an annular groove (9), and the key (3) having at its end a ring (10) which rotates in this groove, the stubs (11) and the notches (12) being formed on said raised element and said ring (10), **characterized in that** said ring (10) is elastically deformable in a plane transverse to the axis of rotation of the key, and **in that** the area (C) where the indexing means are found, has a reduced diameter relatively to the area (B) into which open said apertures.

2. The cock according to claim 1, said ring (10) of which is divided into arc sectors which are separated by cuts (13) and in which the stubs or notches are formed.

3. The cock according to claim 2, said ring (10) of which is divided into two identical sectors (10a, 10b) each of which including a stub (11).

4. The cock according to claim 2, said ring (10) of which is divided into four identical sectors (10c, 10d, 10e, 10f) each of which including a stub (11).

5. The cock according to claim 3 or 4, said raised element (8) of which is conformed laterally to form eight notches (12).

6. The cock according to any of claims 1 to 5, the housing (3) of which has a cross-section which decreases from said end of the housing, and the key (1) has a general frustro-conical shape.

7. The cock according to any of claims 1 to 6, wherein said raised element (8) has a regular profile consisting of a succession of convex sectors (8a) alternating with concave sectors (8b) forming said notches.

8. The cock according to any of claims 1 to 7, the key (1) of which is axially blocked in the housing (3) by penetration of an annular rib (13) formed on the key into a groove (14) formed in the side wall of the housing.

9. The cock according to any of preceding claims, the ring of which has a cross-section which is circular in the undeformed state and which becomes elliptical in the elastically deformed state.

10. The cock according to any of the preceding claims, wherein, when the ring (10) is elastically deformed, a play (20) exists between the convexities (8a) of the raised element (8) and the ring and a play (21) exists between the ring and the wall of the housing of the barrel which is facing the ring.

## Patentansprüche

1. Hahn mit drehbarem Schlüssel, wobei der Schlüssel (1) sich in einer rohrförmigen Aufnahme (3) eines Kegels (2) dreht, in den der Schlüssel axial an einem Ende der Aufnahme bis in eine axiale Position eingeführt wurde, die von Arretiermitteln (13, 14) bestimmt wird, wobei die Seitenwand der Aufnahme Durchgänge (4, 5) umfasst, die in einen Bereich (B) der Aufnahme an Öffnungen einmünden, während der Schlüssel mit einem oder mehreren Durchgängen (6) versehen ist, um diese Öffnungen zu erschließen oder eine Verbindung zwischen bestimmten Öffnungen in Abhängigkeit von Betriebspositionen des Schlüssels im Laufe einer Rotation des Schlüssels herzustellen, wobei der Hahn in einem weiteren Bereich (C) der Aufnahme Mittel, genannt Indexierungsmittel, umfasst, um taktil dem Bediener anzugeben, dass der Schlüssel in einer Betriebsposition angekommen ist, in der er eine Verbindung herstellt, wobei diese Indexierungsmittel Vorsprünge (11) und Einbuchtungen (12) umfassen, die über Seiten gegenüber des Schlüssels und der Aufnahme verteilt sind, damit im Verlauf einer Rotation des Schlüssels die Vorsprünge in die Einbuchtungen eindringen, wenn der Schlüssel in einer Betriebsposition ankommt und daraus nur durch eine spürbare Anstrengung herauskommen kann, die auf den Schlüssel ausgeübt wird, um ihn zum Drehen zu bringen, wobei der Schlüssel oder der Kegel derart ausgelegt sind, dass sie den Vorsprüngen erlauben, durch elastische Wirkung der Einbuchtungen in Folge dieser Anstrengung herauszukommen, wobei die Aufnahme (3) einen geschlossenen Boden (7) umfasst, der ein Relief (8) aufweist, das zwischen ihm und der Seitenwand der Aufnahme eine Ringnut (9) festlegt, wobei der Schlüssel (3) an seinem Ende einen Kranz (10) aufweist, der sich in der Nut dreht, wobei die Vorsprünge (11) und die Einbuchtungen (12) auf dem Relief und auf dem Kranz (10) gebildet sind,
**dadurch gekennzeichnet, dass** der Kranz (10) elastisch deformierbar in einer Ebene quer zur Rotationsachse des Schlüssels ist, dass der Bereich (C), in dem sich die Indexierungsmittel befinden, einen reduzierten Durchmesser bezüglich des Bereichs (B) hat, in dem die Öffnungen einmünden.

2. Hahn gemäß Anspruch 1, wobei der Kranz (10) in Kreisbogensektoren eingeteilt ist, die durch Schnitte (13) getrennt sind und in denen die Vorsprünge oder die Einbuchtungen gebildet sind.

3. Hahn gemäß Anspruch 2, wobei der Kranz (10) in zwei identische Sektoren (10a, 10b) eingeteilt ist, von denen jeder einen Vorsprung (11) umfasst.

4. Hahn gemäß Anspruch 2, wobei der Kranz in vier identische Sektoren (10c, 10d, 10e, 10f) eingeteilt ist, von denen jeder einen Vorsprung (11) umfasst.

5. Hahn gemäß Anspruch 3 oder 4, wobei das Relief (8) seitlich angepasst ist, um acht Einbuchtungen (12) zu bilden.

6. Hahn gemäß einem der Ansprüche 1 bis 5, wobei die Aufnahme (3) einen Querschnitt hat, der von dem Ende der Aufnahme abnimmt, und der Schlüssel (1) eine allgemeine kegelförmige Form hat.

7. Hahn gemäß einem der Ansprüche 1 bis 6, bei welchem das Relief (8) ein regelmäßiges Profil hat, das von einer Folge von konvexen Sektoren (8a) gebildet wird, die mit konkaven Sektoren (8b) abwechseln, die die Einbuchtungen bilden.

8. Hahn gemäß einem der Ansprüche 1 bis 7, dessen Schlüssel (1) axial in der Aufnahme (3) arretiert wird durch Eindringen einer ringförmigen Rippe (13), die auf dem Schlüssel gebildet ist, in eine Nut (14), die in der Seitenwand der Aufnahme gebildet ist.

9. Hahn gemäß einem der vorhergehenden Ansprüche, dessen Kranz einen Querschnitt hat, der im nicht-deformierten Zustand kreisförmig ist und der im elastisch deformierten Zustand elliptisch wird.

10. Hahn gemäß einem der vorhergehenden Ansprüche, bei welchem, wenn der Kranz (10) elastisch deformiert wird, ein Spiel (20) zwischen den Konvexitäten (8a) des Reliefs (8) und der Krone existiert sowie ein Spiel (21) zwischen dem Kranz und der Wand der Aufnahme des Kegels, der gegenüber dem Kranz ist.
